# EUROPÄISCHE PATENTSCHRIFT

(11) **EP 1 244 445 B1**
(45) Veröffentlichungstag und Bekanntmachung des Hinweises auf die Patenterteilung: **31.08.2005**
(21) Anmeldenummer: 00983295.7
(22) Anmeldetag: 12.12.2000
(51) Int. Cl.: A61K 31/4164, A61P 35/00

(54) **VERWENDUNG VON AZOLEN ZUR PRÄVENTION VON STRAHLEN-INDUZIERTEM HAUTKREBS**
USE OF AZOLES FOR PREVENTING SKIN CANCER DUE TO RADIATION
UTILISATION D'AZOLES DANS LA PREVENTION DU CANCER DE LA PEAU CAUSE PAR LA RADIATION

(30) Priorität: 24.12.1999 DE 19963052
(43) Veröffentlichungstag der Anmeldung: 02.10.2002
(73) Patentinhaber: Bayer HealthCare AG, 51368 Leverkusen (DE)
(72) Erfinder: FLADUNG, Bernward, 51519 Odenthal (DE); ENZMANN, Harald, 42111 Wuppertal (DE); Dr.Petersen-Braun, Marianne, 53639 Königswinter (DE)
(86) Internationale Anmeldenummer: PCT/EP2000/012595
(87) Internationale Veröffentlichungsnummer: WO 2001/047505

(56) Entgegenhaltungen:
- WO-A-99/26611
- US-A- 5 633 274
- US-A- 5 998 394
- MUKHTAR H ET AL: "CLOTRIMAZOLE AN INHIBITOR OF EPIDERMAL BENZO-A-PYRENE METABOLISM AND DNA BINDING AND CARCINOGENICITY OF THE HYDROCARBON" CANCER RESEARCH, Bd. 44, Nr. 10, 1984, Seiten 4233-4240, XP001033544 ISSN: 0008-5472
- VANUYTSEL L ET AL: "KETOCONAZOLE THERAPY FOR ADVANCED PROSTATIC CANCER FEASIBILITY AND TREATMENT RESULTS" JOURNAL OF UROLOGY, Bd. 137, Nr. 5, 1987, Seiten 905-908, XP001033554 ISSN: 0022-5347
- PAUL C ET AL: "CHROMOBLASTOMYCOSIS WITH MALIGNANT TRANSFORMATION AND CUTANEOUS-SYNOVIAL SECONDARY LOCALIZATION THE POTENTIAL THERAPEUTIC ROLE OF ITRACONAZOLE" JOURNAL OF MEDICAL AND VETERINARY MYCOLOGY, Bd. 29, Nr. 5, 1991, Seiten 313-316, XP001033552 ISSN: 0268-1218
- KHALID M HUMAYUN ET AL: "Effects of clotrimazole on the growth, morphological characteristics, and cisplatin sensitivity of human glioblastoma cells in vitro." JOURNAL OF NEUROSURGERY, Bd. 90, Nr. 5, Mai 1999 (1999-05), Seiten 918-927, XP001033555 ISSN: 0022-3085

## Beschreibung

Die Erfindung betrifft die Verwendung von Azolen zur Prävention von Hautkrebs, der durch Strahlen hervorgerufen wird.

UV-Strahlen werden je nach Wellenlänge in UV-A-Strahlen (320-400 nm, UV-A-I: 340-400 nm, UV-A-II: 320-340 nm) oder UV-B-Strahlen (280-320 nm) eingeteilt. Ganz allgemein gilt: Die schädigende Wirkung der UV-Strahlen auf die menschliche Haut steigt mit sinkender Wellenlänge und steigender Dauer der Exposition.

UV-Strahlen können akute und chronische Hautschädigungen bewirken, wobei die Art der Schädigung von der Wellenlänge der Strahlung abhängt. So kann die UV-B-Strahlung einen Sonnenbrand (Erythem) bis hin zu schwersten Hautverbrennungen verursachen. Auch Minderungen von Enzymaktivitäten, Störungen der DNS-Struktur und Veränderungen an der Zellmembran als schädigende Wirkung der UV-B-Strahlen sind bekannt. Die UV-A-Strahlen dringen in tiefere Hautschichten ein und können dort den Alterungsprozeß der Haut beschleunigen. Die kürzerwellige UV-A-II-Strahlung verstärkt zusätzlich die Bildung von Sonnenbrand. Außerdem kann die UV-A-Strahlung phototoxische oder photoallergische Hautreaktionen auslösen.

Sehr häufige und ungeschützte Bestrahlung der Haut mit Sonnenlicht kann in extremen Fällen zu krankhaften Hautveränderungen bis hin zum Hautkrebs führen.

Bei der Bekämpfung von Tumoren wird oftmals ionisierende Strahlung, insbesondere Röntgenstrahlung, eingesetzt ("Strahlentherapie"). Dabei wird nicht nur das betroffene Organ, sondern notgedrungen auch die Haut einer Strahlenbelastung ausgesetzt, die schädigend wirkt und im schlimmsten Fall Hautkrebs induzieren kann. Ein Mittel zur Prävention solcher Strahlenschäden wäre überaus wünschenswert.

Azole hemmen das Wachstum von normalen und von Krebszellen in vitro und Tumorwachstum in vivo; vgl. L.R. Benzaquen et al., J.A. (1995) Nat. Med. 1, 534 bis 540.

H Mukhtar et al. (Cancer Research, Vol. 44, No. 10, 1984, S. 4233-4240) beschreiben die Fähigkeit von Clotrimazol, den epidermalen Metabolismus von polycyclischen aromatisierten Kohlenwasserstoffe wie beispielsweise Benzo(a)pyren zu inhibieren. Polycyclische aromatisierte Kohlenwasserstoffe zählen zu chemischen Noxen, die zum Beispiel bei topischer Applikation Krebs auslösen können. Es wird gezeigt, dass eine Inhibierung des Metabolismus chemischer Noxen das Krebsrisiko senken kann.

US 5,998,394 beschreibt die Verwendung von Dihydroxyvitamin D3 [1,25(OH)2D3] zur Behandlung von Hautstörungen und Hautkrankheiten wie beispielsweise Schuppenflechte oder aktinischer Keratose, die in Zusammenhang mit einer Hyperoroliferation. Hypodifferenziation oder Entzündung von epidermalen Zellen stehen. Dabei wird Ketoconazol als 24-Hydroxylase-Inhibitor verwendet, um den Abbau von 1,25(OH)2D3 zu verhindern.

US 5,633,274 beschreibt die Behandlung von nicht Hormon-abhängigen Krebsarten, wie beispielsweise Hautkrebs, durch Imidazote, wie beispielsweise Clotrimazol, Miconazol oder Econazol.

C. Paul et al. (J. Med. Veterin. Mycology, Vol. 29, No.5, 1991, S. 313-316) beschreiben die Behandlung eines Patienten, der an einer chronischen Pilzinfektion (Chromoblastomykose) leidet, mit Itraconazol. Aufgrund der Chronizität trat begleitend eine chronische Entzündung auf, die maligne entartet war. Durch Gabe von hohen Dosen Itraconazol konnte der Patient geheilt werden.

M Humayun Khalid et al. (J. Neurosurpery, Vol. 90, No. 5, May 1999, S. 918-927) beschreiben die antiproliferativen Eigenschaften und die morphologische Beeinflussung von Zellen im Zusammenhang mit Glioblastomzellreihen. Danach wirkt Clotrimazol inhibierend auf das Wachstumsverhalten von stark entarteten, malignen Hirntumoren.

EP-A 0 843 995 offenbart die Verwendung von N-substituierten Benzazolen, welche einen breiten UV-Absorptionsbereich besitzen, zur Herstellung von Sonnenschutzmitteln.

Es wurde nun gefunden, dass sich Azole zur Prävention von Strahlen-induziertem Hautkrebs eignen. "Strahlen-induziert" im Sinne der Erfindung bedeutet vornehmlich "UV-induziert" und "durch Strahlentherapie induziert".

Die Erfindung gestattet es beispielsweise, Azol-haltige Sonnenschutzmittel herzustellen, die die UV-induzierte Entstehung von Hautkrebs, insbesondere von Plattenepithelkarzinomen, Basaliomen und malignen Melanomen, hemmen oder ganz verhindern.

Gegenstand der Erfindung ist also die Verwendung von Azolen zur Herstellung topischer Mittel zur Prävention von Strahlen-induziertem Hautkrebs.

Bevorzugte Azole zur Prävention von Hautkrebs entsprechen beispielsweise der Formel 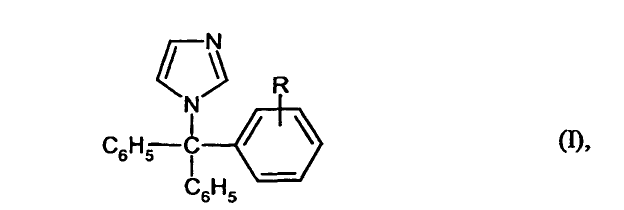 worin
- R: einen Trifluormethyl-, Methoxy- oder o-Chlorsubstituenten bedeutet, vgl. DE-AS 16 70 976.

### Andere bevorzugte Azole umfassen z.B.

- Bifonazol =: 1-(4-Phenylbenzhydryl)-imidazol
- Butoconazol =: (±)-1-[4-(4-Chlorphenyl)-2-[(2,6-dichlorphenyl)thio]butyl]-1-H-imidazol
- Croconazol =: 1-(1-[2-(3-Chlorbenzyloxy)phenyl]vinyl)imidazol
- Clotrimazol =: 1-[(2-Chlorphenyl)-diphenylmethyl]-1H-imidazol
- Econazol =: 1-[2-(4-Chlorbenzyloxy)-2-(2,4-dichlorphenyl)-ethyl]-imidazol
- Fenticonazol =: 1-[2-(2,4-Dichlorphenyl)-2[[4-phenylthio)phenyl]methoxy]-ethyl]-1H-imidiazol
- Fluconazol =: 2-(2,4-Difluorphenyl)-1,3-bis(1H-1,2,4-triazol-1-yl)-2-propanol
- Isocanazol =: 1-[2-(2,4-Dichlorphenyl)-2[(2,6-dichlorphenyl)methoxy]-ethyl]-1H-imidiazol
- Itraconazol =: (±)-2-sec-Butyl-4-[4-(4-{[(2R,4S)-2-(2,4-dichlorphenyl)-2-(1H,1,2,4-triazol-1-ylmethyl)-1,3-dioxolan-4-yl-methoxy]-phenyl}-piperazino)-phenyl]-2,4-dihydro-3H-1,2,4-triazol-3-on
- Ketoconazol =: (±)-1-Acetyl-4-{4-[2α-(2,4-dichlorphenyl)-2β-(1-imidazolylmethyl)-1,3-dioxolan-4β-ylmethoxy]-phenyl}-piperazin
- Miconazol =: (±)-1-[2-(2,4-Dichlorbenzyloxy)-2-(2,4-dichlorphenyl)-ethyl]-1H-imidazol
- Omoconazol =: (Z)-1-[2-[2-(4-Chlorphenoxy]-2-(2,4-dichlorphenyl)-1-methylethenyl]-1H-imidazol
- Oxiconazol =: (Z)-1-(2,4-dichlorphenyl)-2-(1H-imidazol-1-yl)ethanon-O-[(2,4-dichlorphenyl)methyl]oxim
- Sertaconazol =: (±)-1-[2,4-Dichlor-β-[(7-chlorbenzol[b]thien-3-yl)methoxy]-phenethyl]imidiazol
- Sulconazol =: 1-[2-[[(4-Chlorphenyl)methyl]thio]-2-(2,4-dichlorphenyl)ethyl]-1H-imidazol

- Terconazol =: cis-1-[4-[[2-(2,4-Dichlorphenyl)-2-(1H-1,2,4-triazol-1-ylmethyl)-1,3-dioxolan-4-yl]methoxy]phenyl]-4-(1-methylethyl)piperazin
- Tioconazol =: (±)-1-[2-(2-Chlor-3-thienylmethoxy)-2-(2,4-dichlorphenyl)-ethyl]-1H-imidazol

Die erfindungsgemäßen Mittel können in den üblicherweise verwendeten Anwendungsformen vorliegen, d.h. z.B. als Öl-in-Wasser- oder Wasser-in-Öl-Emulsion, als Milch, als Lotion, Creme, Aerosol, Gel.

Die Mittel können üblicherweise verwendete Bestandteile, wie z.B. Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester (z.B. Isopropylpalmiat, Isooctylstearat, Adipinsäurediisopropylester usw.), natürliche oder synthetische Öle und Wachse, Pigmente (z.B. Titandioxid, Zinkoxid, Perglanzpigmente, Farbpigmente), Verdickungsmittel (z.B. Hydroxyethylcellulose, Bentonit usw.), Konservierungsstoffe, UV-Absorber, Feuchtigkeitsmittel, Siliconöle, Vitamine, Glycerin, Ethylalkohol, Parfumöle enthalten.

Die Azole werden im allgemeinen in Mengen von 0,3 bis 30, vorzugsweise 0,5 bis 12, insbesondere 1 bis 6 Gew.-%, bezogen auf die fertige Zubereitung (Mittel), eingesetzt.

Die erfindungsgemäßen Mittel können vor der Strahlenexposition auf die Haut aufgetragen und eingerieben werden. Bei längerer Bestrahlungsdauer, z.B. beim Sonnenbaden, empfiehlt es sich, diesen Vorgang jeweils nach 2 bis 3 Stunden zu wiederholen. Nach innigem Kontakt mit Wasser (Baden, Duschen) sollte die Haut vollständig abgetrocknet und mit dem erfindungsgemäßen Mittel erneut eingerieben werden, falls die Strahlenexposition fortgesetzt werden soll.

### Test der Wirksamkeit

Induktion von Hauttumoren durch UV-Bestrahlung und deren Verminderung an transgenen Mäusen:
- Gruppe 1:: UV-Exposition + gegebenenfalls Sonnenschutz
- Gruppe 2:: UV-Expositionen + Azol + gegebenenfalls Sonnenschutz

- Endpunkt:: Papillome erwartungsgemäß nach 4 bis 12 Wochen
(Entwicklung von Karzinomen erfordert ca. 10 Monate)

Die Resultate der beiden Gruppen zeigen, dass Azole vor UV-induzierten Hauttumoren schützen.

## Patentansprüche

1. Verwendung von Azolen, ausgewählt aus Azolen der Formel 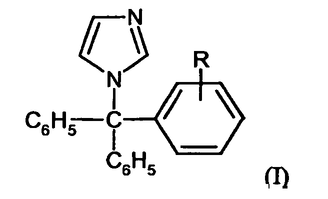 worin R einen Trifluormethyl-, oder Methoxysubstituenten bedeutet, Bifonazol, Butoconazol, Clotrimazol, Croconazol, Econazol, Fenticonazol, Fluconazol, Isoconazol, Itraconazol, Ketoconazol, Miconazol, Omoconazol, Oxiconazol, Sertaconazol, Sulconazol, Terconazol, Tioconazol und deren Mischungen, zur Herstellung von topischen Mitteln zur Prävention von Strahlen-induziertem Hautkrebs.

2. Verwendung gemäß Anspruch 1, **dadurch gekennzeichnet, dass** die topischen Mittel als Öl-in-Wasser-Emulsion, Wasser-in-Öl-Emulsion, Milch, Lotion, Creme, Aerosol oder Gel vorliegen.

3. Verwendung gemäß Anspruch 1 oder 2, **dadurch gekennzeichnet, dass** die topischen Mittel vor der Strahlenexposition auf die Haut aufgetragen werden.

## Claims

1. Use of azoles chosen from azoles of the formula 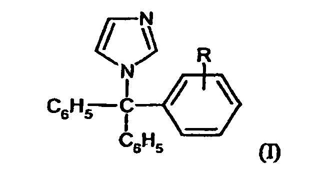 in which R is a trifluoromethyl or methoxy substituent, bifonazole, butoconazole, clotrimazole, croconazole, econazole, fenticonazole, fluconazole, isoconazole, itraconazole, ketoconazole, miconazole, omoconazole, oxiconazole, sertaconazole, sulconazole, terconazole, tioconazole and mixtures thereof for preparing topical compositions for preventing radiation-induced skin cancer.

2. Use according to Claim 1, **characterized in that** the topical compositions are in the form of an oil-in-water emulsion, water-in-oil emulsion, milk, lotion, cream, aerosol or gel.

3. Use according to Claim 1 or 2, **characterized in that** the topical compositions are applied to the skin prior to radiation exposure.

## Revendications

1. Utilisation d'azoles choisis entre des azoles de formule 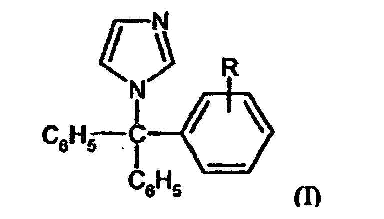 dans laquelle R représente un substituant trifluorométhyle ou méthoxy, le bifonazole, le butoconazole, le clotrimazole, le croconazole, l'éconazole, le fenticonazole, le fluoconazole, l'isoconazole, l'itraconazole, le kétoconazole, le miconazole, l'omoconazole, l'oxyconazole, le sertaconazole, le sulconazole, le terconazole, le tioconazole et leurs mélanges, pour la préparation de compositions topiques destinées à la prévention du cancer cutané causé par irradiation.

2. Utilisation suivant la revendication 1, **caractérisée en ce que** les compositions topiques se présentent sous forme d'émulsion huile-dans-eau, d'émulsion eau-dans-huile, de lait, de lotion, de crème, d'aérosol ou de gel.

3. Utilisation suivant la revendication 1 ou 2, **caractérisée en ce que** les compositions topiques sont appliquées sur la peau avant l'exposition à des rayons.
